# FASCICULE DE BREVET EUROPEEN

(11) **EP 2 689 236 B1**
(45) Date de publication et mention de la délivrance du brevet: **04.03.2015**
(21) Numéro de dépôt: 12711151.6
(22) Date de dépôt: 22.03.2012
(51) Int. Cl.: G01N 21/64

(54) **METHODE CYTOLOGIQUE UTILISANT L'AUTOFLUORESCENCE DES GLOBULES BLANCS POUR LE DIAGNOSTIC PRECOCE ET LE SUIVI DES INFECTIONS**
ZYTOLOGISCHES VERFAHREN UNTER VERWENDUNG VON AUTOMATISCHER FLUORESZENZ WEISSER BLUTKÖRPERCHEN ZUR FRÜHDIAGNOSE UND ÜBERWACHUNG VON INFEKTIONEN
CYTOLOGICAL METHOD USING THE AUTO FLUORESCENCE OF WHITE CORPUSCLES FOR THE EARLY DIAGNOSIS AND THE MONITORING OF INFECTIONS

(30) Priorité: 22.03.2011 FR 1152356
(43) Date de publication de la demande: 29.01.2014
(73) Titulaire: Université de Nantes, 44000 Nantes (FR); UNIVERSITE PARIS-SUD 11, 91400 Orsay (FR); Centre National de la Recherche Scientifique (CNRS), 75016 Paris (FR); CHU Nantes, 44000 Nantes (FR)
(72) Inventeur: ASEHNOUNE, Karim, 44000 Nantes (FR); FONTAINE-AUPART, Marie-Pierre, 94260 Fresnes (FR); LECART, Sandrine, 91190 Gif-sur-Yvette (FR); MONSEL, Antoine, 92320 Châtillon (FR); ROQUILLY, Antoine, 44100 Nantes (FR)
(74) Mandataire: Regimbeau
(86) Numéro de dépôt international: PCT/EP2012/055127
(87) Numéro de publication internationale: WO 2012/127003

(56) Documents cités:
- KIM YEONG SIC ET AL: "Neutrophils with Toxic Granulation Show High Fluorescence with Bis(Zn2+-dipicolylamine) Complex", ANNALS OF CLINICAL AND LABORATORY SCIENCE, vol. 39, no. 2, avril 2009 (2009-04), pages 114-119, XP009150143, ISSN: 0091-7370

## Description

### ART ANTERIEUR

Le sepsis sévère ou infection bactérienne grave reste une cause majeure de morbi-mortalité hospitalière, plus précisément dans les services de soins intensifs ou de réanimation. Son incidence actuelle de 3 à 11 cas pour 1000 habitants aux États-Unis a d'ailleurs augmenté de 8,7% par an ces dix dernières années. 14,6 % des admissions dans les réanimations françaises sont en rapport avec cette pathologie. Malgré de nombreux progrès dans les procédures diagnostiques et thérapeutiques, la mortalité hospitalière liée au sepsis sévère est estimée à 35 % en France. Il est actuellement clairement démontré que le délai séparant l'admission des patients de l'initiation de l'antibiothérapie constitue un facteur pronostique majeur de mortalité. Le but est donc, au 21^{ème} siècle, de diagnostiquer l'infection le plus précocement possible, ce qui est un gage de survie des patients.

En outre, l'un des enjeux actuels en médecine est de pouvoir réduire la masse globale d'antibiotiques utilisés quotidiennement afin de combattre les effets néfastes de leur emploi massif (résistance bactérienne et coûts économiques). La durée des traitements antibiotiques des infections bactériennes est actuellement au centre des principaux débats. Des techniques rapides de monitorage de l'infection en cours et donc de l'efficacité des traitements anti-infectieux employés sont ainsi nécessaires. Ces techniques permettront d'arrêter les traitements au moment adapté et seront à l'origine d'un raccourcissement de la durée des traitements antibiotiques. Par là, elles entraîneront une réduction de la consommation mondiale d'antibiotiques.

Or, les techniques microbiologiques classiques actuellement disponibles que constituent l'examen direct et la culture sur milieux nutritifs, ne permettent pas l'accès à une réponse rapide. En effet, 24 à 48 heures sont nécessaires à l'obtention d'une réponse diagnostique bactériologique. De même, les techniques d'amplification génique (RT-PCR par exemple) sont très chères car elles font appel à des appareils sophistiqués et ne sont pas encore complètement validées dans une indication diagnostique.

Pour remplacer ces techniques microbiologiques ou génétiques, il existe donc un besoin urgent de développer des méthodes fiables, et peu coûteuses, permettant de diagnostiquer des infections de manière assez précoce dans leur développement, et ce de façon beaucoup plus rapide que ce qui est permis actuellement.

Or il est connu que la réponse de l'hôte aux micro-organismes se met en place, dès les premières minutes de l'infection : elle est en fait quasi immédiate. Les globules blancs, cellules clef de l'immunité innée, jouent un rôle fondamental et très précoce dans la reconnaissance et la destruction des agents pathogènes. Ces mécanismes de défense de l'hôte contre les microbes (bactéries et virus) sont sous la dépendance de récepteurs spécifiques, les récepteurs Toll-like (TLRs). Plusieurs de ces TLRs jouent un rôle primordial dans la reconnaissance des bactéries : le TLR4, par exemple, reconnaît des composants de la membrane des bactéries à gram-négatif comme le lipopolysaccharide (LPS), tandis que le TLR2 reconnait des éléments des bactéries à gram-positif comme le peptidoglycane (PGN) Les TLR3, 7 et 8 sont des récepteurs endosomiaux qui reconnaissent les ARN des virus. Une fois stimulés, ces TLR ont pour caractéristique commune d'entraîner l'activation du facteur de transcription nucléaire NF-κB et la production de cytokines pro-inflammatoires NF-κB dépendantes, comme le Tumor Necrosis Factor α (TNF α) et l'interleukine 6 (IL-6). Par ailleurs, une autre voie de signalisation constitue un mécanisme de défense important de l'immunité innée : la voie du récepteur au N-formyl-L-methionyl-L-leucyl-L-phenylalanine (fMLP). Ce peptide, produit de la dégradation de protéines bactériennes, active un récepteur couplé à des protéines G entraînant une cascade d'activation de kinases intra-cytoplasmiques aboutissant à la phosphorylation des sous-unités dont l'assemblage forme la NADH-oxydase. Ce complexe enzymatique membranaire permet aux monocytes et aux Neutrophiles Polynucléaires (PNN) de produire des espèces réactives de l'oxygène (ERO) en oxydant le NADH en NAD. Ces ERO participent à l'activité bactéricide des phagocytes.

Les présents Inventeurs montrent ici qu'il est possible d'utiliser, dans des conditions expérimentales bien particulières, l'autofluorescence de ces globules blancs pour révéler l'activité immunitaire précoce qui se met en place lors de l'infection microbienne (et donc l'état infectieux d'un individu). La méthode diagnostique de l'invention utilise un matériel optique de routine permettant de travailler dans des domaines de longueurs d'onde compatibles avec l'autofluorescence cellulaire, et constitue ainsi une aide rapide, fiable et peu coûteuse au diagnostic d'une infection chez un individu. Elle permet également de contrôler très rapidement l'efficacité des thérapeutiques anti-infectieuses mises en place au préalable.

### RESUME DE L'INVENTION

La présente invention concerne en premier lieu une méthode *in vitro* pour diagnostiquer l'état infectieux d'un individu à partir d'un échantillon de globules blancs issu d'un prélèvement biologique dudit individu, comprenant au moins les étapes suivantes :
i) Mesurer l'intensité cellulaire moyenne de l'autofluorescence dudit échantillon, et
ii) Comparer l'intensité mesurée à l'étape i) avec une valeur contrôle, de manière à déterminer l'état infectieux dudit individu.

Dans un mode de réalisation particulier, les globules blancs dudit échantillon sont choisis parmi les monocytes et/ou les neutrophiles polynucléaires. Dans ce mode de réalisation de l'invention, lorsque l'état dudit individu est infectieux, ledit individu est atteint d'une infection bactérienne, virale ou fongique, de préférence bactérienne.

De préférence, ledit prélèvement biologique est un fluide issu d'un organe potentiellement infecté dudit individu, de manière encore plus préférée ce prélèvement est un fluide pulmonaire, un liquide d'ascite, un fluide céphalo-rachidien, un prélèvement sanguin ou tout autre fluide biologique issu d'un organe potentiellement infecté.

Dans un mode de réalisation préféré, l'intensité de l'autofluorescence des cellules dudit échantillon est mesurée sur cellules fixées chimiquement, au moyen d'un microscope de fluorescence, d'un cytomètre de flux, d'un spectrofluorimètre ou de tout autre dispositif optique capable de mesurer la fluorescence.

Dans un mode de réalisation préféré entre tous, l'échantillon de cellules est préparé en monocouche sur lame de matière transparente, de préférence de verre, au préalable de l'étape i), selon une méthode comportant au moins les étapes suivantes :
a) déposer des cellules dudit échantillon dans au moins un système de cytocentrifugation,
b) centrifuger le dispositif obtenu à l'étape a), de préférence à environ 600 rpm pendant environ 5 minutes, de manière à projeter les cellules sur ladite lame,
c) fixer les cellules ainsi projetées sur la lame pour former un spot cellulaire, avec au moins une goutte de paraformaldéhyde (PFA) 4% environ, de préférence pendant 10 minutes environ,
d) rincer le spot cellulaire précédemment fixé, de préférence avec du tampon PB S,
e) laisser sécher le spot cellulaire précédemment rincé,
f) ajouter sur le spot cellulaire précédemment séché au moins une goutte d'un milieu de montage compatible avec l'observation de la fluorescence,
g) apposer une lamelle couvre-objet transparente, de préférence en verre, sur le spot cellulaire issu de l'étape f).

Lorsque les globules blancs dudit échantillon sont majoritairement des monocytes, l'état dudit individu est infectieux si l'intensité de l'autofluorescence mesurée à l'étape i) est significativement supérieure à la valeur contrôle.

Lorsque les globules blancs dudit échantillon sont majoritairement des polynucléaires neutrophiles, l'état dudit individu est infectieux si l'intensité de l'autofluorescence mesurée à l'étape i) est significativement inférieure à la valeur contrôle.

### LEGENDES DES FIGURES

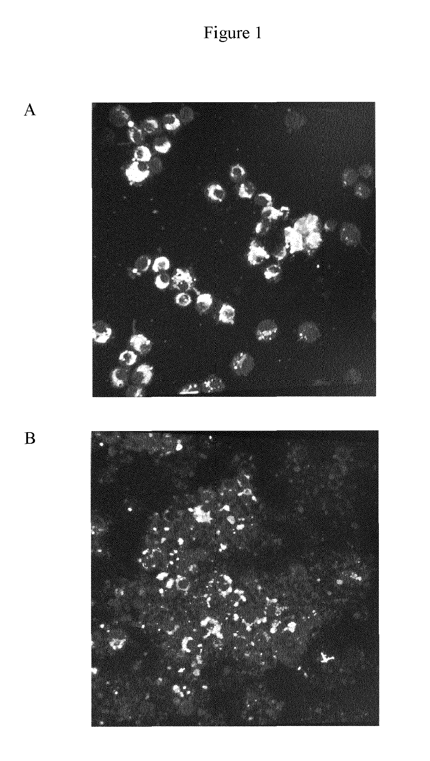

La Figure 1 est constituée de deux images (A, B) prises par un microscope confocal, révélant l'autofluorescence de monocytes et polynucléaires neutrophiles issus de lames cytologiques de lavages broncho-alvéolaires (LBA) dans un modèle murin de pneumonie à *Staphylococcus aureus.*

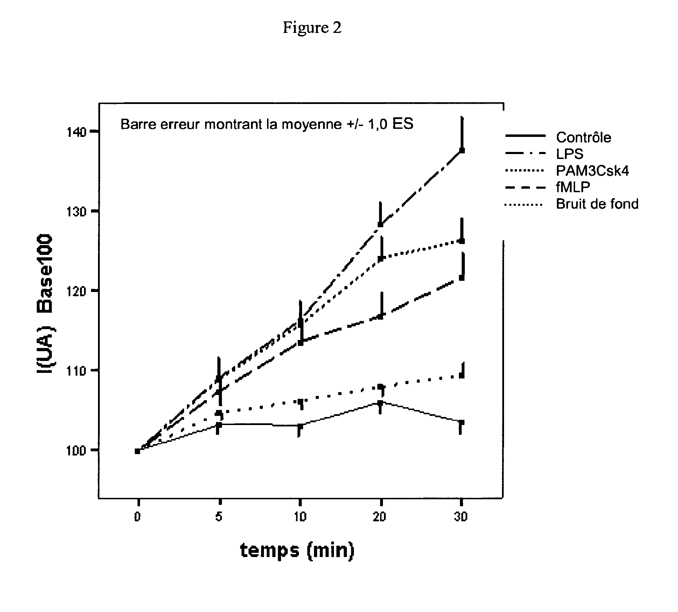

La Figure 2 représente les variations de l'intensité d'autofluorescence (If) en fonction du temps des monocytes humains selon différentes conditions de stimulations.

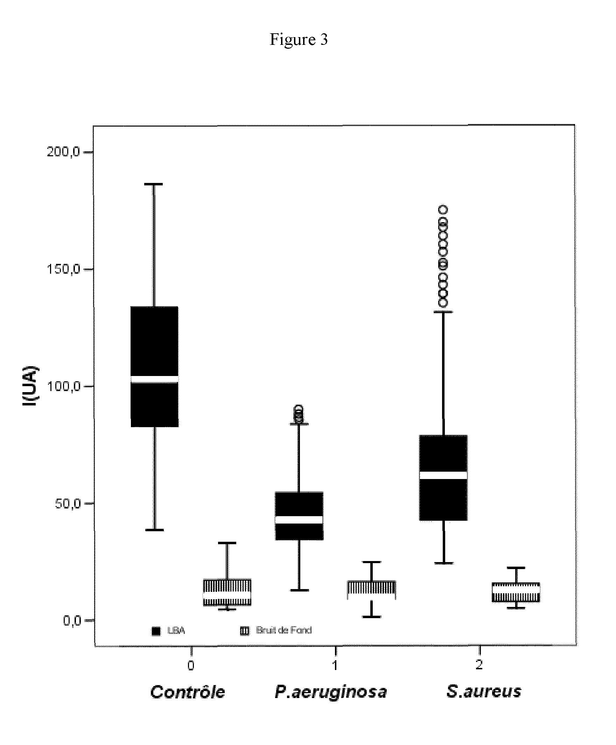

La Figure 3 illustre l'intensité de l'autofluorescence (If) des monocytes-macrophages et PNN prélevées dans les lavages bronchoalvéolaires (LBA, en noir) dans des modèles murins de pneumonies à *Pseudomonas aeruginosa* ou *Staphylococcus aureus*. Le bruit de fond est représenté par des barres.

### DESCRIPTION DETAILLEE DE L'INVENTION

La méthode de la présente invention utilise l'autofluorescence de cellules particulières du système immunitaire en vue de diagnostiquer la présence d'agents bactériens et/ou viraux chez un individu à risque. Les pathogènes infectieux bactériens et/ou viraux sont capables, *via* l'activation des TLRs, de provoquer une activité métabolique précoce chez ces cellules immunitaires. Cette activité métabolique pourrait être corrélée à des variations de concentration du NAD(P)H, l'un des coenzymes essentiels impliqués dans le métabolisme énergétique cellulaire, caractérisé par son autofluorescence intracellulaire (Mayevsky A et al, Am J. Physiol. Cell Physiol. 2007).

Ce signal fluorescent endogène, habituellement gênant dans le cadre d'études utilisant des sondes fluorescentes, est déjà exploité dans le domaine de la cancérologie et du métabolisme cellulaire, son étude permettant de discriminer des états métaboliques divers (US 6,289,236, Palmer GM et al, Photochem Photobiol 2003 ; Li BH et al, World J. Gastroenterol. 2006). Toutefois, aucun test diagnostique utilisant l'autofluorescence de cellules du système immunitaire, et en particulier celles identifiées par les présents Inventeurs, n'a jamais été décrit ni suggéré.

Dans ce domaine, seules deux études ont été réalisées : d'une part l'équipe de Petty et ses collaborateurs ont démontré l'oscillation temporelle et spatiale du signal d'autofluorescence dans des polynucléaires neutrophiles stimulés par des agonistes immunitaires, en essayant de caractériser différents états d'activation de ces cellules (Petty H.R. et al, PNAS 2001; 98 :3145-3149). Cette étude rappelle que l'autofluorescence des neutrophiles et des monocytes est majoritairement due à la présence du NADH qui subit des oscillations temporelles et des déplacements intracellulaires importants, qui peuvent être induits par des signaux d'activation bactérienne. Il est important de noter que le signal étudié est un signal de variation ondulatoire de l'intensité d'autofluorescence dans le temps et l'espace sur une échelle temporelle de 0.1 µs à 20 µs, et sur une échelle spatiale de quelques microns. Les modifications ondulatoires spatio-temporelles ont donc lieu dans un intervalle temporel infinitésimal succédant à la stimulation immune *in vitro* des PNN (quelques µs après stimulation par l'agoniste immunitaire concerné). Ces résultats découragent donc d'utiliser ces fluctuations d'autofluorescence, trop brèves dans le temps et trop localisées dans l'espace, pour une méthode diagnostique. En effet, dans la perspective d'une méthode diagnostique, il est nécessaire que l'état métabolique modifié depuis le temps T1 de l'infection soit stable dans le temps et quantifiable au moment T2 du prélèvement de l'échantillon. L'intervalle de temps séparant la stimulation de cellules d'intérêt et la mise en évidence de l'état métabolique activé représentant en pratique quelques heures à plusieurs jours, il n'est donc absolument pas compatible avec les échelles de temps décrites dans Petty et al.

La deuxième étude concerne la mise en évidence de l'effet du tabac sur l'autofluorescence des macrophages du poumon. Pauly et al ont en effet identifié que le tabagisme augmentait l'intensité de cette autofluorescence (Pauly J.L. et al, Microsc Res Tech 2005).

Par ailleurs, la demande de brevet internationale WO 99/50642 décrit une méthode pour diagnostiquer des infections, basée sur l'exploitation de l'autofluorescence de plasma entier de patients subissant une infection virale (virus du sida ou virus de l'hépatite) pour établir des caractéristiques spectrales d'émission discriminant le plasma des patients malades de ceux des témoins. La méthode de la présente invention exploite non pas l'autofluorescence du plasma (comme le propose WO99/50642), mais l'autofluorescence de cellules précises, à savoir des cellules immunitaires (et, de préférence, parmi ces cellules, les monocytes et les polynucléaires neutrophiles) qu'elles soient issues de sang total ou d'organes potentiellement infectés. Yeong et al., Ann. Clin. Lab. Sci. 39(2), 2009, 114-119 suggère une méthode in vitro pour diagnostiquer l'état infectieux d'un individu basée sur la détermination de la fluorescence des neutrophiles en présence de bis(Zn2+-dipicolylamine), qui forme un complexe fluorescent avec le phosphate du NADP. Les présents Inventeurs ont mis au point pour la première fois un protocole expérimental permettant d'exploiter l'autofluorescence des globules blancs, et en particulier les modulations de cette autofluorescence liées à leur activation. Ils proposent ainsi un système reproductible, fiable et efficace pour évaluer l'état infectieux d'individus cibles.

Dans un premier aspect, la présente invention concerne donc une méthode *in vitro* pour diagnostiquer l'état infectieux d'un individu à partir d'un échantillon de cellules immunitaires issues d'un prélèvement biologique dudit individu, ladite méthode comprenant au moins les étapes suivantes :
i) Mesurer l'intensité cellulaire moyenne de l'autofluorescence dudit échantillon, et
ii) Comparer l'intensité mesurée à l'étape i) avec une valeur contrôle, de manière à déterminer l'état infectieux dudit individu.

Dans le cadre de la présente invention, lorsque l'état d'un individu est « infectieux », ledit individu est infecté par un micro-organisme, de préférence par une bactérie, un virus ou un champignon (levure ou filament). De manière encore plus préférée, ledit individu subit une infection bactérienne. Cet état peut avoir été déjà diagnostiqué auparavant et l'individu peut être soumis à un traitement anti-infectieux (antibiotique). Dans ce cas, la méthode diagnostique de l'invention est destinée à évaluer si l'infection est toujours présente et/ou si on peut arrêter le traitement.

Par « individu » on entend ici un animal ou un mammifère, et en particulier l'homme.

Un prélèvement « biologique » est défini dans la présente invention comme tout prélèvement de fluide biologique, de préférence issu d'organes potentiellement infectés et contenant donc des globules blancs. Il est possible dans le cadre de la présente invention de prélever par exemple un échantillon de fluide pulmonaire (dans le cas de pneumonies), d'ascite (dans les infections de liquide d'ascite), de liquide céphalo-rachidien (dans les méningites) ou de sang, ou de tout autre organe potentiellement infecté.

Au sens de la présente invention, « obtenir un échantillon de cellules issu d'un prélèvement biologique» consiste à 1) prélever un échantillon de fluide biologique d'un individu, de préférence d'un organe d'un individu, et 2) spécifiquement dans le cadre de prélèvement sanguin, purifier à partir de cet échantillon les cellules d'intérêt par les méthodes classiques de biologie cellulaire, pour obtenir l'échantillon de cellules purifiées.

Il est à noter que l'étape optionnelle de purification cellulaire pour l'obtention préférentielle des cellules d'intérêt n'est pas nécessaire lorsqu'un fluide biologique autre que le sang est utilisé. En effet, pour les autres fluides prélevés (fluide pulmonaire, liquide céphalo-rachidien ou ascite), en cas d'infection, ces fluides contiendront en grandes majorité des cellules d'intérêt (PNN et monocytes), et il n'y aura donc pas besoin de les purifier.

Dans le cas d'un prélèvement sanguin, le volume prélevé est de préférence d'au moins 10 mL - 20 mL, de sorte à obtenir suffisamment de cellules d'intérêt après purification. Dans le cas d'un prélèvement d'un fluide issu d'un organe quelques mL suffisent (500 µL à 1 mL par puits de cytospin).

L'échantillon de cellules utilisable dans la méthode diagnostique de l'invention peut être soit conservé à température ambiante pour être utilisé de façon extemporanée (en pratique, moins de 5 heures après le prélèvement), soit conservée à la température de 4°C afin de préserver l'intégrité des cellules jusqu'aux opérations de purification/isolement cellulaire, fixation et/ou de mesure de l'intensité de l'autofluorescence.

Au sens de la présente invention, «l'intensité cellulaire moyenne de l'autofluorescence » mesurée à l'étape i) de la méthode diagnostique de l'invention est l'intensité totale de l'autofluorescence pour une suspension cellulaire ou une valeur moyenne cellulaire obtenue sur au moins environ 50 cellules, de préférence environ 100 cellules. Il convient alors 1) de soustraire à cette valeur totale la valeur de l'autofluorescence due à des éventuelles cellules contaminatrices, et 2) de diviser cette intensité restante par le nombre de cellules d'intérêt (monocytes ou polynucléaires neutrophiles) présentes dans l'échantillon étudié. Cette valeur finale sera l'intensité cellulaire moyenne au sens de la présente invention.

De nombreux logiciels permettent aujourd'hui d'obtenir instantanément une quantification des niveaux d'autofluorescence cellulaires et de faire la moyenne pour un grand nombre de cellules (par exemple, Métamorph, ImageJ, Imaris ou tout autre logiciel de traitement d'image connu de l'homme du métier).

La valeur « contrôle » (ou « standard ») à laquelle il convient de comparer l'intensité de l'autofluorescence de l'individu à tester est l'intensité de l'autofluorescence moyenne cellulaire obtenue à partir d'un grand nombre de cellules isolées provenant de plusieurs individus sains ne prenant aucun traitement depuis au moins 7 jours (notamment ni antibiotique, ni anti-inflammatoire), ne présentant aucune infection détectable déclarée (aucun signe / symptôme infectieux, tel que par exemple : fièvre, courbatures, douleurs, etc.). Cette valeur contrôle est typiquement calculée sur au moins 100 cellules isolées à partir de au moins 5 individus sains. Elle a été calculée au préalable en utilisant les paramètres expérimentaux standards qui seront utilisés pour le patient testé (notamment en termes de matériel optique utilisé, mode de fixation des cellules, longueurs d'onde d'excitation, d'émission, température, pH, etc.).

Les cellules immunitaires, appelées aussi « globules blancs » ou « leucocytes », sont des cellules du sang humain contenant un noyau mono- ou plurilobé, qui jouent essentiellement un rôle dans la défense de l'organisme contre les agents étrangers au sein de l'immunité innée. Parmi les globules blancs, on distingue les cellules mononucléaires (lymphocytes B, lymphocytes T, monocytes, et macrophages) des polynucléaires (ou « granulocytes », que sont les neutrophiles, les éosinophiles, et les basophiles) (Keneth M. Murphy, Paul Travers, & Mark Walport, Janeway's immunobiology. 7th Edition)

De manière préférée, dans le cadre de la présente invention, les globules blancs dudit échantillon sont choisis parmi les monocytes et/ou les neutrophiles polynucléaires.

Dans une variante de la méthode diagnostique de l'invention, l'échantillon cellulaire contient de préférence au moins environ 80%, de préférence environ 90 % de PNN, de manière encore plus préférée au moins environ 95% de PNN

Certains fluides biologiques prélevés directement dans des organes sont connus pour ne contenir que des PNN. Par exemple, les fluides de lavage bronchoalvéolaires ainsi que l'ascite contiennent majoritairement des PNN (E.Pilly, Maladies infectieuse et tropicales, 22ème édition, 2010). Dans ce cas une étape de purification préalable ne sera pas nécessaire.

De préférence, l'échantillon de fluide biologique utilisé dans la présente invention ne subit aucune étape de purification car il contient naturellement au moins environ 80%, de préférence environ 90 % de PNN, de manière encore plus préférée au moins environ 95% de PNN. L'échantillon de fluide biologique est donc préférentiellement un échantillon de LBA, d'ascite ou de Liquide Céphalo-rachidien (LCR).

Néanmoins, si une étape de purification venait à être requise pour obtenir un échantillon de cellule utilisable dans la méthode de l'invention (notamment dans le cas d'un échantillon sanguin), il est préférable d'isoler en premier lieu les cellules polynucléées sanguines, puis, par des techniques connues de l'homme du métier, comme par exemple la sélection par séparation sur colonne magnétique (anti-CD16) obtenir les polynucléaires neutrophiles. Pour obtenir un échantillon de cellules polynucléées à partir d'un fluide biologique, il convient d'utiliser l'une des techniques bien connue de l'homme du métier. A titre d'exemple, on peut citer la technique du gradient de densité puis prélever l'anneau inférieur, qui correspond essentiellement aux cellules polynucléaires (cf technique Cederlane^{®}, Tebu-bio, décrite dans les exemples ci-dessous).

La plupart des pneumonies présentent des formules majoritairement neutrophiliques (PNN majoritaires). Cependant, certaines sont à majorité monocytaires ou panachées (50/50). Ces proportions dépendent de l'agent pathogène responsable de la pneumonie, de l'individu et/ou du stade plus ou moins avancé de la pneumonie. Tous les fluides biologiques désignés dans l'invention (sang, fluide pulmonaire, liquide céphalo-rachidien ou ascite) peuvent contenir des monocytes de façon majoritaire dans certains cas particuliers d'infection.

Dans une autre variante de la méthode diagnostique de l'invention, l'échantillon cellulaire contient donc de préférence au moins environ 80%, de préférence environ 90 % de monocytes, de manière encore plus préférée au moins environ 95% de monocytes.

De préférence, l'échantillon de fluide biologique utilisé dans la présente invention ne subit aucune étape de purification car il contient naturellement au moins environ 80%, de préférence environ 90 % de monocytes, de manière encore plus préférée au moins environ 95% de monocytes (dans le cas de fluide pulmonaire, liquide céphalo-rachidien ou ascite).

Si une étape de purification venait à être requise (l'échantillon est par exemple un échantillon de sang), les monocytes peuvent être isolés à partir des cellules mononuclées par des techniques bien connues de l'homme du métier. A titre d'exemple, on peut effectuer une sélection positive par séparation sur colonne magnétique (anticorps anti-CD14). Pour obtenir un échantillon de cellules mononuclées (lymphocytes, monocytes et macrophages) à partir d'un fluide biologique, il est préférable d'utiliser des techniques de biologie cellulaire bien connues de l'homme du métier. A titre d'exemple, on peut utiliser la technique du gradient de densité, puis prélever l'anneau supérieur qui est constitué de ces cellules (cf technique Cederlane®, Tebu-bio, décrite dans les exemples ci-dessous).

La fluorescence est la propriété que possède une molécule d'émettre un photon pour retourner de son état excité (suite à l'absorption d'un autre photon) à son état fondamental. Les cellules contiennent des molécules qui peuvent devenir fluorescentes lorsqu'elles sont excitées avec des rayons de longueurs d'onde dans le visible ou dans l'ultra-violet (U.V.) Cette émission de fluorescence, émanant de fluorophores endogènes, est une propriété intrinsèque des cellules appelée « auto-fluorescence » ou « fluorescence endogène» et doit être distinguée des signaux de fluorescence obtenus par l'ajout de marqueurs exogènes. Les fluorophores connus sont : les acides aminés aromatiques, les pigments lipophiles, le NADPH, les flavines et les porphyrines. L'autofluorescence ne nécessite pas de marquage spécifique (Monici M., Biotechnol Annu Rev. 2005).

La source lumineuse imposée par la méthode peut donc prendre n'importe quelle forme d'outil optique tant qu'elle est capable de fournir une intensité lumineuse suffisamment puissante dans le domaine de longueur d'onde concerné. Si les sources LASER UV (argon) sont capables de fournir un faisceau incident avec de telles caractéristiques, d'autres outils optiques capables de fournir des gammes de longueur d'ondes compatibles avec l'excitation du NADH cellulaire peuvent également être utilisés. C'est le cas de certaines lampes (mercure, halogène etc.) de diode LASER et de LASER blanc (délivrant un spectre d'émission continu dans une large gamme de longueur d'onde).

Pour révéler et mesurer l'autofluorescence biologique, il est possible d'utiliser 1) des cellules en suspension, i.e. des cellules qui ne sont pas liées à un support, et évoluent librement dans un milieu liquide approprié, ou 2) des cellules fixées sur un support transparent, de préférence une lame de verre.

Selon un mode particulier de l'invention, l'intensité de l'autofluorescence des cellules dudit échantillon est mesurée sur des cellules en suspension. Dans ce mode particulier, l'autofluorescence peut être mesurée avec des systèmes mesurant la fluorescence sans qu'un observateur puisse voir ces cellules, par exemple par cytométrie de flux, par spectrophotométrie, ou par microspectrofluorimétrie.

Dans ce cas, la mesure de l'intensité moyenne de l'autofluorescence se fait automatiquement par des systèmes (cytomètres de flux, spectrophotomètres) mesurant la fluorescence totale de chaque échantillon.

Dans un autre mode particulier de l'invention, l'intensité de l'autofluorescence des cellules dudit échantillon est mesurée sur des cellules fixées chimiquement et placées sur une lame compatible avec l'observation de la fluorescence, de préférence une lame transparente en verre, et l'autofluorescence est alors mesurée avec des appareils de microscopie de fluorescence conventionnelle (microscope à épifluorescence), de microscopie confocale ou biphotonique.

La mesure de l'intensité moyenne de l'autofluorescence se fait alors au moyen d'un logiciel de traitement d'image, celle-ci étant obtenue à partir d'un dispositif adapté tel une caméra couplée à un microscope.

Sans vouloir s'y limiter, on peut utiliser, dans le cadre de la présente invention, un cytomètre de flux, un spectrophotomètre classique, ou tout type de microscope permettant d'obtenir un signal de fluorescence en imagerie d'intensité ou de comptage de photon.

Dans un mode de réalisation préféré de l'invention, l'intensité de l'autofluorescence est mesurée pour des cellules fixées sur une lame transparente en verre, à l'aide d'un microscope à épifluorescence conventionnel.

Dans un mode de réalisation encore plus préféré de l'invention, l'intensité de l'autofluorescence des cellules dudit échantillon est mesurée à l'aide d'un microscope à épifluorescence excitant les cellules avec une longueur d'onde allant de 300 nm environ à 600 nm environ, de préférence allant de 350 nm environ à 450 nm environ. Cette excitation est réalisée de préférence en utilisant une source laser UV.

Par ailleurs, l'intensité de l'autofluorescence des cellules dudit échantillon mesurée à l'étape i) de la présente invention est de préférence celle émise à une longueur d'onde allant de 400 nm environ à 700 nm environ, de préférence allant de 450 nm environ à 600 nm environ.

Dans un mode de réalisation très particulier, l'intensité de l'autofluorescence des cellules dudit échantillon est mesurée à l'étape i) de la méthode diagnostique de l'invention à l'aide d'un microscope à fluorescence confocal utilisant une source laser Argon UV continu, un photomultiplicateur sensible dans le domaine de longueur d'onde allant de 400 nm environ à 550 nm environ, un pinhole ouvert et un objectif X63/1.4 à immersion à huile.

Dans un mode de réalisation de l'invention, la mesure de l'intensité cellulaire moyenne de l'autofluorescence s'effectue sur des cellules vivantes.

De manière préférentielle, ces cellules vivantes ont été isolées à partir d'un organe, et ont été remises en suspension de préférence en milieu liquide dans un dispositif de culture transférable sur un outil optique d'exploitation (comme le puits de culture cellulaire ou LabTek^{®} employé dans la première phase expérimentale ci-dessous décrite).

Dans ce cas, les présents Inventeurs ont démontré qu'il était important, pour mesurer de façon fiable et reproductible l'autofluorescence sur des cellules vivantes, de maintenir le pH et la température du milieu de culture à une valeur constante (pH de préférence compris entre 7 et 8, de manière encore plus préférée valant 7,4, et température maintenue à 37°C) pendant toute la durée de la mesure de l'autofluorescence. Dans le cas contraire, les valeurs de l'intensité de l'autofluorescence mesurées seront moins fiables et moins reproductibles.

Cependant, dans un mode de réalisation préféré de l'invention, l'intensité de l'autofluorescence des cellules de l'échantillon est mesurée sur des cellules fixées chimiquement, c'est-à-dire figées dans un état métabolique cellulaire particulier, par exemple à l'aide de paraformaldéhyde.

Dans ce mode de réalisation, les cellules du prélèvement biologique sont fixées chimiquement le plus vite possible, de préférence moins de 5 heures, après le prélèvement de l'échantillon biologique. En effet, en fixant les globules blancs dans un certain état, c'est un instantané métabolique cellulaire qui est étudié. Ceci permet de s'affranchir de toutes les difficultés techniques liées à la pérennisation d'un état vivant cellulaire qu'imposerait une méthode d'étude sur cellules vivantes. C'est pourquoi il est important de minimiser le temps entre la fixation des cellules de l'échantillon et le prélèvement de l'échantillon. On notera le cas particulier des prélèvements sanguins, dans lequel l'étape de purification ne peut avoir lieu que sur cellules vivantes et donc avant toute étape de fixation.

Les présents Inventeurs ont testé plusieurs modes de fixation, et plusieurs systèmes de montage sur lame afin d'obtenir une mesure fiable de l'autofluorescence pour les deux types de cellules immunitaires (monocytes et PNN, voir l'exemple 3) ci-dessous). Ce faisant, ils ont identifié un protocole commun de traitement de l'échantillon cellulaire adapté aux contraintes de la méthode de l'invention, i.e. qui assure un signal d'autofluorescence suffisant permettant d'obtenir des résultats fiables et reproductibles pour ces deux types cellulaires.

Dans ce mode de réalisation préféré, les cellules doivent être préparées en monocouche par cytocentrifugation sur une lame de microscopie en verre, avant d'être fixées sur ladite lame avec du paraformaldéhyde (PFA), lavées, séchées, puis enfin montées dans un milieu de montage compatible avec l'observation de la fluorescence avant d'être recouvertes d'une lamelle couvre objet en verre.

Plus précisément, dans ce mode de réalisation particulier de la méthode de l'invention, l'échantillon de cellules est préparé en monocouche sur lame de matière transparente, de préférence de verre, au préalable de l'étape i), selon une méthode comportant au moins les étapes suivantes :
a) déposer des cellules dudit échantillon dans au moins un système de cytocentrifugation,
b) centrifuger le dispositif obtenu à l'étape a), de préférence à environ 600 rpm pendant environ 5 minutes, de manière à projeter les cellules sur ladite lame,
c) fixer chimiquement les cellules ainsi projetées sur la lame pour former un spot cellulaire, avec au moins une goutte de paraformaldéhyde (PFA) 4% environ, de préférence pendant 10 minutes environ,
d) rincer le spot cellulaire précédemment fixé, de préférence avec du tampon PBS,
e) laisser sécher le spot cellulaire précédemment rincé,
f) ajouter sur le spot cellulaire précédemment séché au moins une goutte d'un milieu de montage compatible avec l'observation de la fluorescence,
g) apposer une lamelle couvre-objet transparente, de préférence en verre, sur le spot cellulaire issu de l'étape f).

Dans ce mode de réalisation bien particulier, l'étape i) de mesure de l'intensité cellulaire moyenne de l'autofluorescence de l'échantillon se fait à l'aide d'un microscope à fluorescence.

Au cours de l'étape a), le nombre de cellules introduites dans le puits du système de centrifugation est avantageusement compris entre 500 000 à 1 000 000 dans 500µL à 1mL de volume par puits.

Ces cellules sont déposées dans un système de cytocentrifugation de type cytospin (par exemple celui commercialisé par Thermo Electron Corporation sous le nom Shandon cytospin^{R}) qui est constitué d'un puits couplé à une lame de microscope transparente, de préférence en verre.

La cytocentrifugation est une technologie aujourd'hui bien connue qui permet de déposer les cellules d'intérêt mises dans le puits dans une zone bien définie sur une lame de microscope en matière transparente, de préférence en verre, en une monocouche, et permet l'absorption du liquide résiduel par le filtre de la chambre à échantillon. Pendant le fonctionnement de la centrifugeuse, le mouvement rotatoire de l'instrument incline les puits en position droite et centrifuge les cellules sur la zone de dépôt de la lame, fournissant à toutes les cellules la même possibilité de se présenter.

La lame transparente sur laquelle sont déposées les cellules d'intérêt (ou « lame de microscope ») est de préférence une lame de verre adaptée à l'analyse de la fluorescence cellulaire, d'une épaisseur comprise entre 1,2 mm et 1,5 mm, de préférence 1,5 mm, telle que celles commercialisées par Fisher Scientific.

Le dispositif obtenu à l'étape a) est ensuite centrifugé. Cette centrifugation de l'étape b) s'effectue entre 400 et 1000 rpm, de préférence à environ 600 rpm (41g) pendant environ 5 minutes.

Pour réaliser l'étape c), la lame de microscope est ensuite sortie de la centrifugeuse, et le spot cellulaire obtenu après l'étape b) est fixé chimiquement avec une solution de paraformaldéhyde (PFA), diluée dans une solution de, PB S avec une concentration comprise entre 2 et 6 % environ, et étant de préférence de 4% environ.

La quantité de PFA est avantageusement d'environ 15 µL de paraformaldéhyde (PFA) pour 10⁶ cellules environ, ou encore une goutte de 1 mL de PFA sur le spot cellulaire projeté sur la lame.

Le temps de fixation avec le PFA est compris entre 2 minutes et 20 minutes, et est de préférence de 10 minutes environ.

Le spot cellulaire fixé est ensuite rincé, par exemple avec du PB S, pour éliminer le PFA restant (étape d)). 3 lavages sont en général requis afin d'éliminer efficacement le PFA restant.

Au cours de l'étape e), le spot cellulaire est séché complètement, par exemple à l'air pendant le temps nécessaire environ 10 minutes à température ambiante,

Le spot cellulaire est ensuite recouvert d'une goutte (de préférence 20 µl) d'un milieu de montage compatible avec l'observation de la fluorescence. Ce milieu est par exemple le milieu de montage « ProLong^{®} Gold» ou « Fluoromount », ou encore « Vectachield Slow Fade ». De préférence, ce milieu est le « ProLong^{®} Gold».

Enfin, l'échantillon doit ensuite être recouvert d'une lamelle couvre-objet adaptée à la mesure de la fluorescence, de préférence en verre, typiquement de 0,13 à 0,17 mm d'épaisseur, comme celles proposées par FisherScientific.

Les présents Inventeurs ont démontré que, en comparant l'intensité cellulaire moyenne de l'autofluorescence de l'échantillon du patient testé à la valeur dite « contrôle », lorsque les globules blancs dudit échantillon sont majoritairement des monocytes, l'état dudit individu est infectieux si l'intensité de l'autofluorescence mesurée à l'étape i) est significativement supérieure à la valeur contrôle.

En d'autres termes, si un échantillon de cellules comprenant au moins environ 70%, de préférence au moins environ 80%, de manière encore plus préférée au moins environ 90% de monocytes, émet un signal d'autofluorescence significativement plus importante que la valeur contrôle, alors l'individu à qui l'échantillon de fluide biologique a été prélevé est atteint d'une infection (ou est toujours infecté) bactérienne, virale ou fongique.

Le terme « significativement supérieur » signifie dans le cadre de la présente invention que le ratio [intensité moyenne cellulaire du patient] / [valeur contrôle] est au minimum de environ 1,2, avantageusement compris entre 1,3 et 3 environ, de manière préférée compris entre 1,5 et 2 environ.

Les présents Inventeurs ont démontré que, à l'inverse, en comparant l'intensité cellulaire moyenne de l'autofluorescence de l'échantillon du patient testé à la valeur dite « contrôle », lorsque les globules blancs dudit échantillon sont majoritairement des polynucléaires neutrophiles, l'état dudit individu est infectieux si l'intensité de l'autofluorescence mesurée à l'étape i) est significativement inférieure à la valeur contrôle.

La différence de résultat entre les deux populations cellulaires peut trouver son explication, entre autre, dans la nature métabolique très différente de ces deux cellules.

En d'autres termes, si un échantillon de cellules comprenant au moins environ 70%, de préférence au moins environ 80%, de manière encore plus préférée au moins environ 90% de polynucléaires neutrophiles, et émet un signal d'autofluorescence significativement plus faible que la valeur contrôle, alors l'individu à qui l'échantillon d fluide biologique a été prélevé est atteint d'une infection (ou est toujours infecté) bactérienne, virale ou fongique.

Le terme « significativement inférieur » signifie, dans le cadre de la présente invention que le ratio [intensité moyenne cellulaire du patient ] / [valeur contrôle] est au maximum de environ 0,8, avantageusement compris entre 0,1 et 0,7 environ, de manière préférée compris entre 0,1 et 0,6 environ.

L'invention décrit aussi une méthode de préparation d'un échantillon cellulaire fixé destiné à être utilisé dans la méthode de diagnostic telle que décrite ci-dessus, et comportant les étapes suivantes :
a) déposer les cellules dans au moins un système de cytocentrifugation,
b) centrifuger le dispositif obtenu à l'étape a), de préférence à environ 600 rpm soit 41g pendant environ 5 minutes, de manière à projeter des cellules sur ladite lame,
c) fixer chimiquement les cellules ainsi projetées sur la lame, formant un spot cellulaire, avec au moins une goutte de paraformaldéhyde (PFA) 4% environ, de préférence pendant 10 minutes environ,
d) rincer le spot cellulaire précédemment fixé, de préférence avec du tampon PB S,
e) laisser sécher le spot cellulaire précédemment rincé,
f) ajouter sur le spot cellulaire précédemment séché au moins une goutte d'un milieu de montage compatible avec l'observation de la fluorescence, et
g) apposer une lamelle couvre-objet transparente, de préférence en verre, sur le spot cellulaire issu de l'étape f).

Les étapes a) à g) sont telles que décrites ci-dessus.

Avantageusement, cette méthode de préparation pourrait être réalisée de façon automatique au moyen de robots capables d'effectuer ces étapes de façon séquentielle.

Un autre avantage de la méthode diagnostique de l'invention réside dans le fait qu'elle pourrait être réalisée de façon entièrement automatique à partir d'un seul appareil compact :
1) préparation automatique de l'échantillon du patient
2) mesure automatique de l'autofluorescence cellulaire moyenne des échantillons,
3) comparaison des valeurs aux valeurs contrôles,
4) sortie du résultat : présence d'un agent bactérien, viral ou fongique chez ledit patient.

Ce système automatisé a l'avantage d'être beaucoup plus rapide que les techniques microbiologiques actuellement utilisées, et beaucoup moins onéreuse que les techniques génétiques visant à identifier la présence d'un agent infectieux qui de plus restent incomplètement validées notamment dans les cas d'infections bactériennes.

### EXEMPLES

### 1) Réactifs et matériel utilisés

Le sang total a été prélevé dans des tubes BD Vacutainer® de 4,5 mL avec 0,5 mL de citrates de Sodium 3,8% (p/v). Le milieu de culture RPMI 1640 provient du laboratoire Invitrogen® (Paisley, Royaume-Uni).

Le gradient de densité : Lympholyte -Poly^{®} provient du laboratoire Cedarlane Tebu-bio^{®} (Le Perrey-en-Yvelines, France). Le kit MACS^{®} pour isolement des monocytes ainsi que les anticorps anti-CD 14 proviennent des laboratoires Miltenyi biotec^{®} (Paris). Le lipopolysaccharide (LPS) d'Esherishia coli 0111 :B4 purifié provient du laboratoire Sigma^{®} (Saint Louis, Missouri, Etats Unis d'Amérique). Le Pam3Csk4 (ci-après PAM) provient du laboratoire InvivoGen^{®}, (San Diego, Etats Unis d'Amérique). Le fMLP provient du laboratoire Sigma-Aldrich^{®} (St Quentin Fallavier, France). Les chambres d'observation stériles pour microscopie confocale Lab-Tek^{®} proviennent du laboratoire Brands Products^{®} (Etats Unis d'Amérique). Les plaques 24 puits et les tubes coniques stériles proviennent du laboratoire Falcon^{®}, Becton Dickinson Labware (Europe, Le Pont de Claix, France). Le milieu de montage spécifique ProLong^{®} Gold antifade reagent provient du laboratoire Invitrogen (ref : P36934). La centrifugeuse permettant la cyto centrifugation et la projection sur lame des échantillons cellulaires est la Shandon Cytospin^{®} Centrifuge.

Le microscope disponible sur la plateforme d'imagerie du centre de Photonique biomédicale d'Orsay est un microscope confocal TCS SP5^{®} de la marque Leica^{®}. Il dispose de 4 lasers continus (2 lasers Hélium-Néon 633 nm et 543 nm, un laser Argon visible 458 nm, 476 nm, 488 nm et 514 nm et un laser Argon UV 351 nm et 364 nm) et d'un laser Titane/Saphir infrarouge haute cadence impulsionnel. Le premier mode d'acquisition utilisé est celui d'analyse en intensité de fluorescence (I_{f}) quantifié en UA.

### 2) Conditions d'acquisition du signal d'autofluorescence du NAD(P)H

A partir des premières observations cellulaires, les conditions expérimentales optiques optimales donnant le meilleur compromis en terme de résolution d'image d'intensité de l'autofluorescence du NAD(P)H, et de détérioration de l'échantillon biologique ont été fixées.

Pour l'acquisition du signal d'intensité de fluorescence, la source laser Argon UV continue a été utilisée (λ = 364 nm, P = 1,3 mW), ainsi qu'un photomultiplicateur (gain = 1250V) dans le domaine de longueur d'onde allant de 400 nm à 550 nm, avec un pinhole ouvert au 1/3 de l'ouverture maximale, avec un objectif X 63 à immersion à huile. Un moyennage de 4 images avec une résolution de 1024 pixels/1024 pixels pour une taille finale de 246µm/246µm a été appliqué pour l'affichage de l'image en I_{f}.

### 3) Démonstration de l'exploitabilité de l'autofluorescence de cytologie obtenue à partir de monocytes et de PNN humains stimulés ex vivo.

Après information et accord des volontaires, 20 ml de sang total ont été prélevé chez 8 volontaires sains. La population de volontaires sains était constituée d'individus hommes et femmes, âgés de 25 à 60 ans, ne prenant aucun traitement depuis au moins 7 jours, notamment pas d'antibiotiques ni d'anti inflammatoires. Ils ne présentaient pas d'infections apparemment en cours d'évolution. Leur sang a été obtenu par ponction veineuse au pli du coude.

### Pour l'obtention des monocytes :

A partir de chaque échantillon sanguin (20 ml), les CMN (cellules mononuclées) ont été isolées grâce à une technique de gradient de densité bien connue de l'homme du métier (Lympholyte -Poly^{®}, laboratoire Cedarlane Tebu-bio^{®} ,Le Perrey-en-Yvelines, France). Cette technique permet d'obtenir deux anneaux cellulaires distincts, un anneau supérieur de CMN (lymphocytes et monocytes) qui était prélevé et un anneau inférieur de cellules polynucléées, constitué essentiellement de polynucléaires neutrophiles. Les monocytes ont alors été isolés à partir des CMN grâce à une technique de sélection positive par séparation sur colonne magnétique (MACS^{®}, Miltenyi biotec, Paris). Les monocytes sanguins ont ensuite été mis en suspension dans du RPMI 1640 + sérum humain 4% + pénicilline G (100U/ml) et streptomycine (100µg/ml) (Valbiotech).

### Pour l'obtention des PNN :

A partir de chaque échantillon sanguin (12 mL), la centrifugation sur Ficol (40 minutes à 1600 tours / min à +4°C) a permis de récupérer l'anneau inférieur remis en suspension dans du RPMI + Serum humain (4%) + EDTA (5%). Deux lavages étaient ensuite réalisés dans ce milieu (10 minutes à 1600 tours / min à +4°C). Puis une déplétion des éosinophiles par ajout d'un anticorps anti CD16 était réalisée selon une sélection négative par séparation sur colonne magnétique (MACS^{®}, Miltenyi biotec, Paris). Les PNN ont ensuite été mis en suspension dans du RPMI 1640 + sérum humain 4% + pénicilline G (100U/ml) et streptomycine (100µg/ml) (Valbiotech).

Les deux populations cellulaires ainsi obtenues (PNN et monocytes) ont été incubées 90 minutes en présence des agonistes immunitaires avec une densité cellulaire de 10⁶ cellules/mL. Le LPS a été utilisé à une concentration finale de 50 µg/mL, le PAM à une concentration finale de 10 µg/mL, le fMLP à une concentration finale de 10⁻⁶ mol/L.

Après 90 minutes, un volume de 500 µL d'échantillon cellulaire (soit 5.10⁵ cellules) a été délicatement prélevé et déposé dans un puits couplé à une lame de cytologie selon le dispositif de confection cytospin^{®}. Un cycle de centrifugation-projection sur lame a été réalisé à 600 RPM, pendant 8 minutes avec une accélération-décélération basse. La fixation du spot cellulaire projeté sur lame a été réalisée par une goutte de paraformaldhéyde (PFA) 4% pendant 10 minutes suivie de 3 rinçages délicats avec une solution tampon type PBS. Après séchage complet, une goutte de 20 µl de milieu de montage ProLong^{®} Gold antifade reagent fraîchement décongelé a été déposée sur le spot cellulaire fixé. Une lamelle couvre-objet en verre a ensuite été délicatement déposée sur la goutte de milieu de montage en faisant en sorte d'éviter toute formation de bulles d'air.

Les lames de cytologies ainsi obtenues ont été alors observées sur microscope confocal à balayage laser. Pour l'acquisition du signal d'intensité de fluorescence, la source laser Argon UV continu a été utilisée (λ = 364 nm, P = 1,3 mW), ainsi qu'un photomultiplicateur (gain = 1250V) dans le domaine de longueur d'onde allant de 400 nm à 550 nm, avec un pinhole ouvert au 1/3 de l'ouverture maximale, et un objectif X 63 à immersion à huile. Un moyennage de 4 images avec une résolution de 1024 pixels/1024 pixels pour une taille finale de 246µm/246µm a été appliqué pour l'affichage de l'image en I_{f}.

Une augmentation significative de l'If de 10% à 18% est constatée pour les monocytes stimulés par rapport aux monocytes contrôles. Une diminution significative de l'If (d'un facteur 2) est par ailleurs constatée pour les PNN stimulés par rapport aux PNN contrôles. Il semble donc exister pour les monocytes stimulés comme pour les PNN stimulés, une différence dans l'If d'autofluorescence par rapport aux monocytes ou PNN contrôles. Cette opposition « PNN-Monocytes » dans le sens de la variation de l'If des cellules stimulées s'explique probablement par les différences fondamentales de métabolisme énergétique cellulaire et les moyens de défenses contre le pathogène.

En ce qui concerne le protocole de cytocentrifugation (cytospin), plusieurs forces de rotation ont été testées : 300 rpm, 600 rpm, 800 rpm, ainsi que plusieurs temps de centrifugation (6 min, 8 min, 10 min). La combinaison de la force 600 rpm avec le temps de 8 minutes s'est avérée la plus performante et la plus rentable en termes de temps de préparation des échantillons cytologiques.

Plusieurs protocoles de fixation des échantillons cellulaires ont été testés, notamment le PFA 4% et le spray alcoolique à l'éthanol (Merckofix Spray, Merck art), ainsi qu'une fixation à -20°C dans de l'acétone ou éthanol ou méthanol. Les meilleurs résultats en termes d'exploitabilité de l'autofluorescence cellulaire ont été constatés avec l'usage du PFA 4%.

Plusieurs modes de montage de l'échantillon cellulaire entre lame et lamelle ont été testés, notamment l'Eukitt Mounting Médium (ProSciTech), le Fluoromount-G (SouthernBiotech), et le Prolong Gold Reagent (Invitrogen, Molecular Probes). Les meilleurs résultats en termes d'exploitabilité de l'autofluorescence des échantillons cellulaires ont été constatés avec le Prolong Gold Reagent.

### 4) Démonstration de l'exploitabilité de l'autofluorescence de cellules de LBA prélevées dans un modèle murin de pneumonie.

En ce qui concerne le modèle de pneumonie à staphylocoque, une souche de *Staphyloccocus aureus* methicilline sensible (SASM) (souche ATCC 29213) a été cultivée 16 heures à 37°C dans un milieu tryptic soja. Immédiatement avant utilisation, les cultures ont été lavées deux fois (centrifugées 10 minutes à 1000 g) et diluées dans du sérum salé isotonique stérile pour être calibrées par spectroscopie. La concentration bactérienne a été systématiquement contrôlée par culture quantitative.

En ce qui concerne le modèle de pneumonie à *Pseudomonas Aeruginosa,* une souche *Pseudomonas Aeruginosa* wild type a été cultivée 18 heures à 37°C en milieu liquide Tryptic Soja. Immédiatement avant l'instillation intra-trachéale, les cultures ont été lavées deux fois (centrifugées 10 minutes à 5000 g à 37°C) et diluées dans du sérum isotonique stérile pour être calibrées pas spectroscopie. Puis calibration par néphélémétrie pour obtenir une concentration à 1.10⁶ UFC/ml. Le contrôle se fait sur gélose Cétrimide (milieu sélectif pour le *Pseudomonas aeruginosa*).

Les souris ont été anesthésiées par Isoflurane, et placées en décubitus dorsal. Une aiguille de nutrition entérale (24 gauges) a été utilisée pour le cathétérisme de la trachée et l'injection de 70 µl de solution bactérienne (staphylocoque ou *Pseudomonas Aeruginosa*). Les souris ont été alors suspendues 30 secondes par les incisives pour améliorer la pénétration de l'inoculum. Le taux d'instillation intra trachéale a atteint 100%.

24 heures après, immédiatement après l'euthanasie, un lavage bronchoalvéolaire a été réalisé en cathétérisant la trachée par abord percutanée (cathéter 24 gauges). Un lavage par 3 x 1 ml de sérum physiologique a alors été réalisé. Un compte cellulaire sur Mallasez a alors été réalisé. Le liquide de lavage alvéolaire a alors été centrifugé (10 minutes à 5000 g à 37°c) et le culot cellulaire a été resuspendu dans du sérum physiologique qsp 1.10⁶ cellules / mL.

Un volume de 500 µL d'échantillon cellulaire (soit 5.10⁵ cellules) a été délicatement prélevé et déposé dans un puits couplé à une lame de microscopie selon le dispositif de confection cytospin^{®}. Un cycle de centrifugation-projection sur lame a été réalisé à 600 RPM, pendant 8 minutes avec une accélération-décélération basse. La fixation du spot cellulaire projeté sur lame a été réalisée par une goutte de paraformaldhéyde (PFA) 4% pendant 10 minutes suivie de 3 rinçages délicats avec une solution tampon type PBS. Après séchage complet, une goutte de 20 µl de milieu de montage ProLong^{®} Gold antifade reagent fraîchement décongelé a été déposée sur le spot cellulaire fixé. Une lamelle de verre a été ensuite délicatement déposée sur la goutte de milieu de montage en faisant en sorte d'éviter toute formation de bulles d'air.

La proportion des différents types cellulaires PNN / macrophages présents dans le liquide de lavage alvéolaire a été caractérisée (marquage Giemsa):

| | **Nombre cellules (/µL)** | **Macrophages (%)** | **Polynucléaires neutrophiles (%)** |
|---|---|---|---|
| Sham | 338 +/- 230 | 67 +/- 5 * | 33 +/- 5 |
| *Staphylococcus aureus* | 343 +/- 161 | 7 +/- 4 | 93 +/- 4 * |
| *Pseudomonas aeruginosa* | 255 +/- 57 | 20 +/- 9 | 80 +/- 9 * |

| | | | |
|---|---|---|---|
| * P < 0.05 *versus* Sham | | | |

Une augmentation très significative du nombre de cellules PNN par rapport aux macrophages est observée chez les souris ayant été inoculées par l'une ou l'autre des solutions bactériennes (staphylocoque ou *Pseudomonas Aeruginosa*). Les pourcentages de polynucléaires neutrophiles observés chez les animaux infectés par rapport aux animaux contrôles (sham) confirment la présence d'une pneumopathie bactérienne.

Les lames d'échantillons ainsi obtenues ont été alors observées sur microscope confocal à balayage laser. Pour l'acquisition du signal d'intensité de fluorescence, la source laser Argon UV continu a été utilisée (λ = 364 nm, P = 1,3 mW), ainsi qu'un photomultiplicateur (gain = 1250V) dans le domaine de longueur d'onde allant de 400 nm à 550 nm, pinhole ouvert au 1/3 de l'ouverture maximale, avec un objectif X 63 à immersion à huile. Un moyennage de 4 images avec une résolution de 1024 pixels/1024 pixels pour une taille finale de 246µm/246µm a été appliqué pour l'affichage de l'image en I_{f}.

29 souris ont été étudiées avec 8 souris contrôle, 9 souris avec pneumonie à *Pseudomonas Aeruginosa,* et 12 souris avec pneumonie à Staphylocoque. L'intensité d'autofluorescence moyenne cellulaire diminue pour les lames issues des souris avec pneumonie par rapport aux cellules issues de LBA de souris contrôles. Les cellules majoritaires (PNN) issus de LBA de souris avec pneumonie fluorescent moins que celles issues de LBA de souris contrôle (44, 8 UA vs 107,5 UA). Un facteur 2 existe entre l'intensité moyenne d'autofluorescence des cellules issues de LBA de souris avec pneumonie et l'intensité moyenne d'autofluorescence des cellules issues de LBA de souris contrôle. Ces résultats permettent de discriminer des cellules issues de LBA de souris contrôle et des cellules issues de LBA de souris atteintes de pneumonie ; cette discrimination se base sur une différence d'intensité moyenne d'autofluorescence. Le constat d'une variation opposée du signal d'autofluorescence des cellules issues de LBA de souris infectées à celui des monocytes dans le modèle de stimulation *ex*-*vivo*, confirme que la majorité des cellules contenues dans les LBA de souris infectées est constituée de PNN. Ce résultat est alors en accord avec la diminution de l'intensité d'autofluorescence des PNN stimulés *ex-vivo* par rapport aux PNN contrôle non stimulés dans le modèle précédent de cytologie de cellules stimulées *ex*-*vivo*. La différence du sens de la variation de l'intensité moyenne d'autofluorescence entre PNN et monocytes stimulés réside probablement dans leurs différences notables en termes de métabolisme énergétique et de moyens de défense contre le pathogène qu'entraine leur stimulation.

### REFERENCES BIBLIOGRAPHIQUES

Babior B.M. et al, Blood 1984 ;64 :959-966
Brun-Buisson C et al. Intensive Care Med 2004,30:580-588.
E.Pilly, Maladies infectieuses et tropicales, 22ème édition, 2010
Keneth M. Murphy, Paul Travers, & Mark Walport, Janeway's immunobiology. 7th Edition (Chapitre 2, Innate Immunity).
Li BH et al, World J. Gastroenterol. 2006 ; 24 :1213-1217
Martin GS et al., N Engl J Med 2003;348:1546-1554.
Mayevsky A et al, Am J. Physiol. Cell Physiol. 2007 ;292 :C615-640
Monici M. , Biotechnol Annu Rev. 2005;11:227-56.
Palmer GM et al, Photochem Photobiol 2003 ;78 :462-469
Pauly J.L. et al, Microsc Res Tech 2005 ;67 :79-89
Petty H.R. et al, PNAS 2001, vol.98, n°6, 3145-3149

## Revendications

1. Méthode *in vitro* pour diagnostiquer l'état infectieux d'un individu à partir d'un échantillon de globules blancs issu d'un prélèvement biologique dudit individu, comprenant au moins les étapes suivantes :
i) Mesurer l'intensité cellulaire moyenne de l'autofluorescence dudit échantillon, et
ii) Comparer l'intensité mesurée à l'étape i) avec une valeur contrôle, de manière à déterminer l'état infectieux dudit individu.

2. Méthode selon la revendication 1, **caractérisée en ce que** les globules blancs dudit échantillon sont choisis parmi les monocytes et/ou les neutrophiles polynucléaires.

3. Méthode selon les revendications 1 ou 2, **caractérisée en ce que**, lorsque l'état dudit individu est infectieux, ledit individu est atteint d'une infection bactérienne, virale ou fongique, de préférence bactérienne.

4. Méthode selon l'une quelconque des revendications 1 à 3, **caractérisée en ce que** ledit prélèvement biologique est un fluide issu d'un organe dudit individu.

5. Méthode selon l'une quelconque des revendications 1 à 4, **caractérisée en ce que** ledit prélèvement biologique est un fluide pulmonaire, un liquide d'ascite, un fluide céphalo-rachidien, un prélèvement sanguin ou un fluide de tout autre organe potentiellement infecté.

6. Méthode selon l'une quelconque des revendications 1 à 5, **caractérisée en ce que** l'intensité de l'autofluorescence des cellules dudit échantillon est mesurée sur des cellules en suspension.

7. Méthode selon la revendication 6, **caractérisée en ce que** l'intensité de l'autofluorescence desdites cellules en suspension est mesurée par cytométrie de flux ou par spectrophotométrie.

8. Méthode selon l'une quelconque des revendications 1 à 5, **caractérisée en ce que** l'intensité de l'autofluorescence des cellules dudit échantillon est mesurée sur des cellules fixées chimiquement et placées sur une lame compatible avec l'observation de la fluorescence, de préférence une lame en verre transparente.

9. Méthode selon l'une quelconque des revendications 1 à 8, **caractérisée en ce que** l'intensité de l'autofluorescence des cellules dudit échantillon est mesurée à l'aide d'un microscope à fluorescence.

10. Méthode selon l'une quelconque des revendications 1 à 5, 8 et 9, dans laquelle ledit échantillon de cellules est préparé en monocouche sur une lame de matière transparente au préalable de l'étape i), selon une méthode comportant au moins les étapes suivantes :
a) déposer des cellules dudit échantillon dans au moins un système de cytocentrifugation,
b) centrifuger le dispositif obtenu à l'étape a), de préférence à environ 600 rpm pendant environ 5 minutes, de manière à projeter les cellules sur ladite lame,
c) fixer chimiquement les cellules ainsi projetées sur la lame pour former un spot cellulaire, avec au moins une goutte de paraformaldéhyde (PFA) 4% environ, de préférence pendant 10 minutes environ,
d) rincer le spot cellulaire précédemment fixé, de préférence avec du tampon PBS,
e) laisser sécher le spot cellulaire précédemment rincé,
f) ajouter sur le spot cellulaire précédemment séché au moins une goutte d'un milieu de montage compatible avec l'observation de la fluorescence,
g) apposer une lamelle couvre-objet transparente, de préférence en verre, sur le spot cellulaire issu de l'étape f).

11. Méthode selon l'une quelconque des revendications précédentes, **caractérisée en ce que**, lorsque les globules blancs dudit échantillon sont majoritairement des monocytes, l'état dudit individu est infectieux si l'intensité de l'autofluorescence mesurée à l'étape i) est significativement supérieure à la valeur contrôle.

12. Méthode selon l'une quelconque des revendications précédentes, **caractérisée en ce que**, lorsque les globules blancs dudit échantillon sont majoritairement des polynucléaires neutrophiles, l'état dudit individu est infectieux si l'intensité de l'autofluorescence mesurée à l'étape i) est significativement inférieure à la valeur contrôle.

## Patentansprüche

1. *In vitro*-Verfahren zum Diagnostizieren des infektiösen Zustandes eines Individuums anhand einer Probe von Leukocyten, die aus einer biologischen Probe des Individuums stammen, mindestens die folgenden Schritte umfassend:
i) Messen der mittleren zellulären Intensität der Autofluoreszenz in der Probe, und
ii) Vergleichen der in Schritt i) gemessenen Intensität mit einem Kontrollwert, um so den infektiösen Zustand des Individuums zu bestimmen.

2. Verfahren nach Anspruch 1, **dadurch gekennzeichnet, dass** die Leukocyten der Probe ausgewählt sind aus Monocyten und/oder mehrkernigen Neutrophilen.

3. Verfahren nach Anspruch 1 oder 2, **dadurch gekennzeichnet, dass**, wenn der Zustand des Individuums infektiös ist, das Individuum eine bakterielle Infektion, eine virale Infektion oder eine Pilzinfektion hat, bevorzugt eine bakterielle Infektion.

4. Verfahren nach einem der Ansprüche 1 bis 3, **dadurch gekennzeichnet, dass** die biologische Probe eine Flüssigkeit ist, die aus einem Organ des Individuums stammt.

5. Verfahren nach einem der Ansprüche 1 bis 4, **dadurch gekennzeichnet, dass** die biologische Probe Lungenflüssigkeit, Ascitesflüssigkeit, zerebrospinale Flüssigkeit, eine Blutprobe oder eine Flüssigkeit aus einem beliebigen anderen Organ ist, das potenziell infiziert ist.

6. Verfahren nach einem der Ansprüche 1 bis 5, **dadurch gekennzeichnet, dass** die Intensität der Autofluoreszenz der Zellen der Probe an Zellen in Suspension gemessen wird.

7. Verfahren nach Anspruch 6, **dadurch gekennzeichnet, dass** die Intensität der Autofluoreszenz der Zellen in Suspension mittels Durchflusscytometrie oder mittels Spektrophotometrie gemessen wird.

8. Verfahren nach einem der Ansprüche 1 bis 5, **dadurch gekennzeichnet, dass** die Intensität der Autofluoreszenz der Zellen der Probe an Zellen gemessen wird, die chemisch fixiert und auf einen für die Fluoreszenzbeobachtung geeigneten Objektträger aufgebracht sind, vorzugsweise auf einen Objektträger aus durchsichtigem Glas.

9. Verfahren nach einem der Ansprüche 1 bis 8, **dadurch gekennzeichnet, dass** die Intensität der Autofluoreszenz der Zellen der Probe mithilfe eines Fluoreszenzmikroskops gemessen wird.

10. Verfahren nach einem der Ansprüche 1 bis 5, 8 und 9, wobei die Zellprobe vor Schritt i) als Monolayer auf einem Objektträger aus einem transparenten Material nach einem Verfahren hergestellt wird, das mindestens die folgenden Schritte umfasst:
a) Einbringen der Zellen der Probe in mindestens ein Zytozentrifugationssystem,
b) Zentrifugieren des in Schritt a) erhaltenen Präparats bevorzugt bei ungefähr 600 UpM während ungefähr 5 Minuten, so dass die Zellen auf den Objektträger aufgetragen werden,
c) chemisches Fixieren der so auf den Objektträger aufgetragenen Zellen, um einen Zellspot zu bilden, mit mindestens einem Tropfen ungefähr 4%-igem Paraformaldehyd (PFA), bevorzugt für etwa 10 Minuten,
d) Spülen des zuvor fixierten Zellspots, vorzugsweise mit PBS-Puffer,
e) Trocknen lassen des zuvor gespülten Zellspots,
f) Hinzufügen mindestens eines Tropfens eines für die Fluoreszenzbeobachtung geeigneten Montagemediums zu dem zuvor getrockneten Zellspot,
g) Auflegen eines transparenten Deckglases, bevorzugt aus Glas, auf den Zellspot aus Schritt f).

11. Verfahren nach einem der vorstehenden Ansprüche, **dadurch gekennzeichnet, dass**, wenn die Leukocyten der Probe überwiegend Monocyten sind, der Zustand des Individuums infektiös ist, wenn die Intensität der in Schritt i) gemessenen Autofluoreszenz signifikant über dem Kontrollwert liegt.

12. Verfahren nach einem der vorstehenden Ansprüche, **dadurch gekennzeichnet, dass** wenn die Leukocyten der Probe überwiegend mehrkernige Neutrophile sind, der Zustand des Individuums infektiös ist, wenn die Intensität der in Schritt i) gemessenen Autofluoreszenz signifikant unter dem Kontrollwert liegt.

## Claims

1. An *in vitro* method for diagnosing the infectious state of an individual from a sample of white blood cells arising from a biological sample from said individual, comprising at least the following steps:
i) measuring the mean cellular autofluorescence intensity of said sample, and
ii) comparing the intensity measured in step i) with a control value, so as to determine the infectious state of said individual.

2. The method according to claim 1, **characterized in that** the white blood cells of said sample are selected from monocytes and/or polymorphonuclear neutrophils.

3. The method according to claim 1 or 2, **characterized in that**, when the state of said individual is infectious, said individual is suffering from a bacterial, viral or fungal infection, preferably bacterial.

4. The method according to any one of claims 1 to 3, **characterized in that** said biological sample is a fluid arising from an organ of said individual.

5. The method according to any one of claims 1 to 4, **characterized in that** said biological sample is a pulmonary fluid, an ascites fluid, cerebrospinal fluid, a blood sample or a fluid of any other potentially infected organ.

6. The method according to any one of claims 1 to 5, **characterized in that** the autofluorescence intensity of the cells of said sample is measured on cells in suspension.

7. The method according to claim 6, caracterized in that the autofluorescence intensity of said cells in suspension is measured by flow cytometry or spectrophotometry.

8. The method according to any one of claims 1 to 5, **characterized in that** the autofluorescence intensity of the cells of said sample is measured on cells that are chemically fixed and placed on a slide compatible with the observation of fluorescence, preferably a transparent glass slide.

9. The method according to any one of claims 1 to 8, **characterized in that** the autofluorescence intensity of the cells of said sample is measured using a fluorescence microscope.

10. The method according to any one of claims 1 to 5, 8 and 9, wherein said sample of cells is prepared in a monolayer on a slide of transparent material prior to step i), according to a method comprising at least the following steps:
a) depositing cells of said sample in at least one cytocentrifugation system,
b) centrifuging the device obtained in step a), preferably at about 600 rpm for about 5 minutes, so as to project the cells on said slide,
c) chemically fixing the cells thus projected on the slide to form a cell spot, with at least one drop of about 4% paraformaldehyde (PFA), preferably for about 10 minutes,
d) rinsing the previously fixed cell spot, preferably with PBS buffer,
e) allowing the previously rinsed cell spot to dry,
f) adding to the previously dried cell spot at least one drop of a mounting medium compatible with the observation of fluorescence,
g) affixing a transparent cover slip, preferably made of glass, on the cell spot arising from step f).

11. The method according to any one of the preceding claims, **characterized in that**, when the white blood cells of said sample are mainly monocytes, the state of said individual is infectious if the autofluorescence intensity measured in step i) is significantly greater than the control value.

12. The method according to any one of the preceding claims, **characterized in that**, when the white blood cells of said sample are mostly polymorphonuclear neutrophils, the state of said individual is infectious if the autofluorescence intensity measured in step i) is significantly lower than the control value.
